# EUROPEAN PATENT APPLICATION

(11) **EP 2 263 661 A1**
(43) Date of publication of application: **22.12.2010**
(21) Application number: 09726142.4
(22) Date of filing: 09.03.2009
(51) Int. Cl.: A61K 9/70, A61K 47/32

(54) **ORAL PREPARATION**

(30) Priority: 28.03.2008 JP 2008088671
(71) Applicant: LINTEC Corporation, Tokyo 173-0001 (JP)
(72) Inventor: SUGIURA, Yusaku, Tokyo 173-0001 (JP); TAKAHASHI, Akira, Tokyo 173-0001 (JP); HIRONAGA, Maki, Tokyo 173-0001 (JP); KABUTO, Akio, Tokyo 173-0001 (JP); SUZUKI, Eiji, Tokyo 173-0001 (JP)
(74) Representative: Solf, Alexander
(86) International application number: PCT/JP2009/054417
(87) International publication number: WO 2009/119290

(57) **Abstract**

An orally-administered agent according to the present invention comprises: a medicine-containing layer containing a medicine and having surfaces; collapse-controlling layers respectively provided on the surfaces of the medicine-containing layer; and gel-forming layers respectively provided on the collapse-controlling layers, wherein the gel-forming layers are swelled and gelatinized by absorbing water to form a gel. The collapse-controlling layers are constituted of a material containing a stomach-soluble material to be dissolved by being in contact with gastric juice. The orally-administered agent according to the present invention can be swallowed with ease and release the medicine in intended parts, in particular, the stomach of a living body.

## Description

### TECHNICAL FIELD

The present invention relates to an orally-administered agent.

### RELATED ART

As examples of an orally-administered agent containing a medicine, there are known solid formulations and jelly-like (or gel-like) semisolid formulations. The solid formulations (e.g., tablets and capsules) are usually hard to take as they are and therefore have to be taken together with a large quantity of water. It is often difficult for aged persons or infants to take the solid formulations. In addition, there are risks that the solid formulations are likely to get stuck in a trachea or may adhere to an esophagus.

In contrast, the jelly-like semisolid formulations are easy to swallow. Therefore, the jelly-like semisolid formulations can be easily taken by aged persons or infants. However, since the semisolid formulations contain a large quantity of moisture, they have a drawback in that the medicine contained therein is susceptible to decomposition or degradation. Moreover, there is a need that the semisolid formulations prevent infiltration of bacteria when producing and storing the semisolid formulations. This makes it cumbersome and complicated to handle the semisolid formulations. For these reasons stated above, such drawbacks prevent the semisolid formulations from being widespread in a market.

In view of the problems noted above, a study has been made in recent years on thin sheet-like (film-like) formulations (see, e.g., the following Patent Document). Within an oral cavity, surfaces of the film formulations are dissolved by saliva or the film formulations are gelatinized by absorbing water. Therefore, it is relatively easy to swallow the film formulations. In addition, there is no need to add water into the film formulations. This makes it easy to handle the film formulations when producing and storing the same.

However, since the film formulations are relatively thin sheet-like formulations, a medicine contained therein is likely to flow out by body fluids such as saliva. Therefore, in the case where such film formulations are used, it is difficult to release a medicine at intended organs of a living body (e.g. intestine and stomach) and make the medicine absorbed into the organs. Furthermore, in the case where the medicine contained in the film formulations flows out into the oral cavity by saliva, there is a case that the medicine may give unpleasant feelings to recipients due to tastes of the medicine itself (e.g., a bitter taste and an astringent taste), senses within the oral cavity by the medicine (e.g., a sense of numbness), odors of the medicine and the like. Therefore, there is a problem in that it is difficult for patients, in particular infants, to follow the compliance of the medicine.

The Patent Document is JP-A 11-116469 as an example of related art.

### SUMMARY OF THE INVENTION

It is an object of the present invention to provide an orally-administered agent that can be swallowed with ease and can release a medicine within a stomach.

Such an object is achieved by the present invention which is described below by the items (1) to (7).
(1) An orally-administered agent comprises: a medicine-containing layer containing a medicine and having surfaces; collapse-controlling layers respectively provided on the surfaces of the medicine-containing layer; and gel-forming layers respectively provided on the collapse-controlling layers, wherein the gel-forming layers are swelled and gelatinized by absorbing water to form a gel; wherein the collapse-controlling layers are constituted of a material containing a stomach-soluble material to be dissolved by being in contact with gastric juice.

(2) In the orally-administered agent described in the above-mentioned item (1), the stomach-soluble material is a stomach-soluble polymer.

(3) In the orally-administered agent described in the above-mentioned item (2), the stomach-soluble polymer is a stomach-soluble acrylic acid-based copolymer.

(4) In the orally-administered agent described in the above-mentioned items (1) to (3), the medicine-containing layer is covered with the collapse-controlling layers.

(5) In the orally-administered agent described in the above-mentioned items (1) to (4), the gel-forming layers contain an anionic polymer.

(6) In the orally-administered agent described in the above-mentioned items (1) to (5), the orally-administered agent has surfaces and surface layers constituting the surfaces, the orally-administered agent further comprising at least one antiadhesive layer as the surface layers, wherein the at least one antiadhesive layer prevents the orally-administered agent from adhering to an inside wall of an oral cavity by dissolving to water.

(7) In the orally-administered agent described in the above-mentioned items (1) to (6), each of the gel-forming layers has a surface provided with a plurality of convex portions.

According to the present invention, it is possible to provide an orally-administered agent that can be swallowed with ease and can release a medicine within a stomach.

### BRIEF DESCRIPTION OF THE DRAWINGS

FIG. 1 is a section view showing an orally-administered agent in accordance with a first embodiment of the present invention.
FIG. 2 is a section view showing an orally-administered agent in accordance with a second embodiment of the present invention.

### DETAILED DESCRIPTION OF THE PREFERRED EMBODIMENTS

Hereinafter, the present invention will be described in detail based on certain preferred embodiments.
An orally-administered agent of the present invention may have any shape. The following description will be made on the assumption that the present orally-administered agent is a film-like formulation (or a sheet-like formulation).

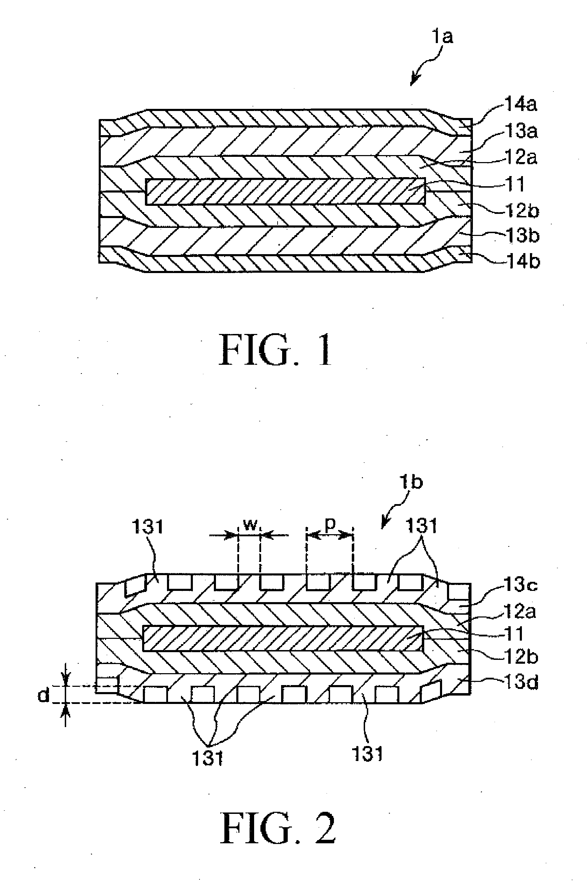

Hereinbelow, embodiments of the present invention will be described with reference to the accompanying drawings.
In the following description, the upper side in FIGs. 1 and 2 will be referred to as "upper" and the lower side thereof will be referred to as "lower" for convenience of explanation.

### <First Embodiment>

First, a description will be made on an orally-administered agent in accordance with a first embodiment of the present invention.

FIG. 1 is a section view showing an orally-administered agent in accordance with a first embodiment of the present invention.
As shown in FIG. 1, the orally-administered agent 1a is configured as a laminated body. Such a laminated body includes a medicine-containing layer 11 which contains a medicine, a collapse-controlling layer 12a which is laminated on an upper surface of the medicine-containing layer 11, a collapse-controlling layer 12b which is laminated on a lower surface of the medicine-containing layer 11, a gel-forming layer 13a which is laminated on an upper surface of the collapse-controlling layer 12a, and a gel-forming layer 13b which is laminated on a lower surface of the collapse-controlling layer 12b. That is, the medicine-containing layer 11 is covered with the collapse-controlling layers 12a and 12b. As shown in FIG. 1, it is preferred that the laminated body further includes an antiadhesive layer 14a which is provided on an upper surface of the gel-forming layer 13a and an antiadhesive layer 14b which is provided on a lower surface of the gel-forming layer 13b as surface layers of the orally-administered agent 1a.

The orally-administered agent 1a is a film-like formulation (or a sheet-like formulation) in a whole shape thereof. Since the orally-administered agent 1a is the film-like formulation, it is possible to reduce a moisture content in the film-like formulation. Furthermore, it is possible to enhance stability of the medicine (especially, an easily-hydrolysable medicine) contained in the medicine-containing layer 11 as compared with a jelly-like formulation which contains a large quantity of moisture. Moreover, the film-like formulation is easy to handle, and it is possible to assist in reducing a packing cost of the film-like formulation.

Furthermore, the gel-forming layers 13a and 13b constituting the surfaces of the orally-administered agent 1a can be swelled and gelatinized within the oral cavity of a patient by water contained in saliva etc. This makes it possible to change a state of the orally-administered agent 1a to a state of it having a size, a shape, elastic force, a viscosity and the like for allowing a patient to swell it with ease. Accordingly, the patient can easily swallow the orally-administered agent 1a. Furthermore, the orally-administered agent 1a has a low risk of getting stuck in a trachea of the patient when swallowing the orally-administered agent 1a. Therefore, even in the case where the patient is aged persons or infants, it is possible to swallow the orally-administered agent 1a safely.

In addition, the orally-administered agent 1a includes the collapse-controlling layers 12a and 12b between the medicine-containing layer 11 and the gel-forming layers 13a and 13b, respectively. The collapse-controlling layers 12a and 12b include a stomach-soluble material which is dissolved by being in contact with gastric juice within a stomach. Accordingly, the collapse-controlling layers 12a and 12b are prevented from dissolving within the oral cavity. Therefore, the medicine contained in the orally-administered agent 1a is prevented from being released within the oral cavity. On the other hand, the collapse-controlling layers 12a and 12b are collapsed by being in contact with the gastric juice within the stomach. As a result, the medicine-containing layer 11 can be dissolved by being in contact with the gastric juice within the stomach to thereby release the medicine contained in the medicine-containing layer 11 in the stomach.

In view of the above, the orally-administered agent 1a exhibits excellent swallowability and can release the medicine at intended parts (in particular, stomach) of the living body.

In this regard, in the present invention, the collapse of the collapse-controlling layers means that a constituent material of the collapse-controlling layers is dissolved and decomposed and then flows out of the collapse-controlling layers, so that the collapse-controlling layers are collapsed.

Hereinbelow, the respective layers constituting the orally-administered agent 1a will be described in more detail.

### <Medicine-Containing Layer>

The medicine-containing layer 11 is a layer containing the medicine to be administered into the intended parts of the living body.

The medicine contained in the medicine-containing layer 11 is a medicine to be administered to the intended parts of the living body of a patient. Such a medicine is not limited to a specific one but may be any orally-administrable medicine. Examples of the orally-administrable medicine include: medicines acting on a central nerve, including a hypnotic medicine such as amobarbital, estazoram, triazolam, nitrazepam, pentobarbital or the like, a psychotropic medicine such as amitriptyline hydrochloride, imipramine hydrochloride, oxazolam, chlordiazepoxide, chlorpromazine, diazepam, sulpiride, haloperidol or the like, an antiparkinson medicine such as trihexyphenidyl, levodopa or the like, an analgesic medicine and an anti-inflammatory medicine such as aspirin, isopropylantipyrine, indometacin, diclofenac sodium, mefenamic acid, streptokinase, streptodornase, serrapeptase, pronase or the like and a central nervous metabolic activation medicine such as ATP, vinpocetine or the like; medicines acting on a respiratory organ, including an expectorant medicine such as carbocysteine, bromhexine hydrochloride or the like and an antiasthmatic medicine such as azelastine hydrochloride, oxatomide, theophylline, terbutaline sulfate, tranilast, procaterol hydrochloride, ketotifen fumarate or the like; medicines acting on a circulatory system, including a cardiac stimulant such as aminophylline, digitoxin, digoxin or the like, an antiarrhythmic medicine such as ajmaline, disopyramide, procainamide hydrochloride, mexiletine hydrochloride or the like, an antianginal medicine such as amyl nitrite, alprenolol hydrochloride, isosorbide dinitrate, nicorandil, oxyfedrine, dipyridamole, dilazep hydrochloride, diltiazem hydrochloride, nitroglycerin, nifedipine, verapamil hydrochloride or the like, a peripheral vasodilator such as kallidinogenase or the like, an antihypertensive medicine such as atenolol, captopril, clonidine hydrochloride, metoprolol tartrate, spironolactone, triamterene, trichlormethiazide, nicardipine, hydralazine hydrochloride, hydrochlorothiazide, prazosin hydrochloride, furosemide, propranolol hydrochloride, enalapril maleate, methyldopa, labetalol hydrochloride, reserpine or the like and an antiarteriosclerotic medicine such as clofibrat, dextran sulfate, nicomol, niceritrol or the like; blood and hematological medicines, including a hemostatic medicine such as carbazochrome sodium sulfonate, tranexamic acid or the like, an antithrombogenic medicine such as ticlopidine hydrochloride, warfarin potassium or the like and an anemia medicine such as ferric sulfate or the like; medicines acting on a gastrointestinal system, including an antiulcer medicine such as azulene, aldioxa, cimetidine, ranitidine hydrochloride, famotidine, teprenone, rebamipide or the like, an antiemetic medicine such as domperidone, metoclopramide or the like, a cathartic medicine such as sennoside, digestive enzyme preparations, and a therapeutic medicine for liver diseases such as glycyrrhizin, liver extract preparations or the like; medicines acting on a metabolic disease, including an antidiabetic medicine such as glibenclamide, chlorpropamide, tolbutamide or the like and an antipodagric medicine such as allopurinol, colchicines or the like; medicines for an ophthalmic field, including acetazolamide; medicines for an otological field, including an anti-vertigo medicine such as difenidol hydrochloride, betahistine mesylate or the like; chemotherapeutic medicines and antibiotic medicines including isoniazid, ethambutol hydrochloride, ofloxacin, erythromycin stearate, cefaclor, norfloxacin, fosfomycin calcium, minocycline hydrochloride, rifampicin, rokitamycin or the like; antineoplastic medicines including cyclophosphamide, tegafur or the like; immunosuppressive medicines including azathioprine; hormones and endocrine medicines including progestational hormone, salivary hormone, thiamazole, prednisolone, betamethasone, liothyronine, levothyroxine or the like; and physiologically active substances (autacoids) including an antihistamine medicine such as diphenhydramine hydrochloride, clemastine fumarate, D-chlorpheniramine maleate or the like and a vitamin such as alfacalcidol, cobamamide, tocopherol nicotinate, mecobalamin or the like. One or more of these medicines may be used independently or in combination according to the purposes of treatment and prevention of a condition.

In particular, in the present invention, the orally-administered agent 1a can release the medicine within the stomach. Therefore, it is preferred that used is a medicine of generating effects by being absorbed in the stomach as the medicine contained in the medicine-containing layer 11.

Furthermore, various kinds of medicines including a medicine administered in a small quantity and a medicine administered in a large quantity can be contained in the medicine-containing layer 11. In this regard, the medicine administered in a small quantity means a medicine whose one-time dosage amount is 1 mg or less, while the medicine administered in a large quantity means a medicine whose one-time dosage amount is 300 mg or more.

A content of the medicine in the medicine-containing layer 11 is not particularly limited and may be suitably adjusted depending on a kind of medicine and the volume of the medicine-containing layer 11. The content of the medicine is preferably in the range of 0.01 to 70 mass%, more preferably in the range of 0.01 to 40 mass% and even more preferably in the range of 0.01 to 35 mass%. This makes it possible to have a sufficiently quantity of the medicine contained in the orally-administered agent 1a while enhancing physical strength of the orally-administered agent 1a.

Furthermore, the orally-administered agent 1a exhibits great enough physical strength even when a relatively large quantity of the medicine as described above is contained in the medicine-containing layer 11 or when an insoluble to water and bulky medicine having a tendency to reduce the physical strength of the medicine-containing layer 11 is contained in the medicine-containing layer 11. Presumably, this is because the collapse-controlling layers 12a and 12b impart great enough physical strength to the orally-administered agent 1a by providing the collapse-controlling layer 12a provided on the upper surface of the medicine-containing layer 11 and the collapse-controlling layer 12b provided on the lower surface thereof in the orally-administered agent 1a.

The medicine-containing layer 11 may include a base (namely, a base agent for the medicine-containing layer) which serves to keep the administered medicine to the intended parts of the living body in a desired state in the medicine-containing layer 11 and to adjust the shape and the physical strength of the medicine-containing layer 11. Examples of the base used in the medicine-containing layer 11 include, but are not limited to: cellulose such as crystalline cellulose, carboxymethyl cellulose, hydroxypropyl cellulose, hydroxypropyl methyl cellulose, methyl cellulose, ethyl cellulose, acetyl cellulose, cellulose acetate phthalate, hydroxypropylmethyl cellulose phthalate, hydroxypropylmethyl cellulose acetate sucinate and carboxymethylethyl cellulose; derivatives of cellulose or pharmaceutically acceptable salts of cellulose (e.g., sodium salt); starch such as α-starch, oxidized starch, carboxymethyl starch sodium, hydroxypropyl starch, dextrin and dextran; derivatives of starch; sugars such as saccharose, maltose, lactose, glucose, fructose, pullulan, xanthane gum, cyclodextrin, xylitol, mannitol and sorbitol; acrylic-acid derivatives such as a dimethylaminoethyl (metha)acrylate-(metha)acrylic acid copolymer, a (metha)acrylic acid-ethyl(metha)acrylate copolymer, a (metha)acrylic acid-methyl(metha)acrylate copolymer, an ethyl(metha)acrylate-chlorotrimethylammonium (metha)acrylic acid copolymer, a dimethylaminoethyl (metha)acrylate-chloromethyl (metha)acrylate copolymer and a (metha)acrylic acid-chloroethyl (metha)acrylate copolymer; Sellac; polyvinylacetal diethylamino acetate; polyvinyl acetate; polyvinyl alcohol; polyvinyl pyrrolidone; a vinylacetate-vinylpyrrolidone copolymer; natural rubbers such as Arabic gum and tragacanth gum; polyglucosamines such as chitin and chitosan; proteins such as gelatin, casein and soybean protein; titanium oxide; calcium monohydrogen phosphate; calcium carbonate; talc; stearic acid; magnesium aluminometasilicate; magnesium silicate; and silicic anhydride. One or more of these bases may be used independently or in combination according to the purposes of including the base into the medicine-containing layer 11.

A content of the base in the medicine-containing layer 11 is not particularly limited, but may be preferably in the range of 30 to 99.9 mass%, more preferably in the range of 60 to 99.9 mass% and even more preferably in the range of 65 to 99.0 mass%. This makes it possible to sufficiently enhance the physical strength of the medicine-containing layer 11 with ease while allowing a sufficiently quantity of the medicine to be contained in the medicine-containing layer 11.

A thickness of the medicine-containing layer 11 can be suitably adjusted within a range permitting an oral administration of the orally-administered agent 1a. The thickness of the medicine-containing layer 11 is not particularly limited, but may be preferably in the range of 0.5 to 5000 µm, more preferably in the range of 10 to 3000 µm and even more preferably in the range of 50 to 1000 µm. This makes it possible to sharply reduce variations in the medicine content and the thickness which would occur in respective portions of the medicine-containing layer 11. In addition, this makes it possible to sufficiently increase overall softness of the orally-administered agent 1a and to greatly enhance ease of swallowing the orally-administered agent 1a.

### <Collapse-Controlling Layer>

The collapse-controlling layer 12a is provided between the medicine-containing layer 11 and the gel-forming layer 13a. Furthermore, the collapse-controlling layer 12b is provided between the medicine-containing layer 11 and the gel-forming layer 13b.

In addition, the collapse-controlling layers 12a and 12b are bonded to each other so as to cover the medicine-containing layer 11. Therefore, the circumference of the medicine-containing layer 11 is covered by the collapse-controlling layers 12a and 12b. This makes it possible for the medicine-containing layer 11 to prevent the medicine contained therein from inadvertently being eluted and altered by being in contact with the body fluids carelessly.

The collapse-controlling layers 12a and 12b are a layer of being capable of collapsing by being in contact with the gastric juice. Therefore, the collapse-controlling layers 12a and 12b are collapsed within the stomach, thereby enabling the medicine-containing layer 11 to be dissolved by being in contact with the gastric juice. Consequently, the medicine is released into the stomach.

Furthermore, the collapse-controlling layers 12a and 12b of the orally-administered agent 1a also have a function of preventing contact between the medicine-containing layer 11 and the saliva. Therefore, the collapse-controlling layers 12a and 12b can prevent the medicine contained in the medicine-containing layer 11 from being dissolved into the oral cavity. This makes it possible to mask a taste of the medicine (e.g. bitter taste and astringent taste), a sense of the oral cavity generated by the medicine (numbness) or an odor of the medicine.

In contrast, in the case where an orally-administered agent does not have such collapse-controlling layers 12a and 12b, there is a fear that water contained in the saliva penetrates from gel-forming layers to a medicine-containing layer so that a medicine flows out into the oral cavity.

Hereinafter, since the collapse-controlling layers 12a and 12b have the same configuration, a description will be made on the collapse-controlling layer 12a as a representative.

In the present invention, the collapse-controlling layer 12a include a stomach-soluble material which is dissolved by being in contact with the gastric juice. By including such a stomach-soluble material, the collapse-controlling layer 12a is in contact with the gastric juice within the stomach and then collapsed.

The stomach-soluble material, which can be used in the collapse-controlling layer 12a, is not particularly limited. For example, a material can be used, which is dissolved under the acid conditions and is difficult to dissolve under the neutral and alkali conditions. Specifically, examples of the stomach-soluble material include: various kinds of inorganic compound such as calcium carbonate and sodium hydrogen carbonate; various kinds of stomach-soluble polymer; and the like. One or more of these compounds may be used independently or in combination.

Examples of the stomach-soluble polymer include: stomach-soluble polyvinyl derivatives such as polyvinyl aminoacetal and polyvinyl acetal diethylaminoacetate; stomach-soluble acrylic acid-based copolymer such as methyl(metha)acrylate-butyl(metha)acrylate-dimethylaminoethyl (metha)acrylate copolymer, and aminoalkyl(metha)acrylate copolymer; and the like.

Among polymers described above, the stomach-soluble material is preferably the stomach-soluble acrylic acid-based copolymer and more preferably methyl(metha)acrylate-butyl(metha)acrylate-dimethylaminoethyl (metha)acrylate copolymer. This makes it possible to enhance the stability of the shape of the collapse-controlling layer 12a. Furthermore, it is possible for the collapse-controlling layer 12a to quickly be dissolved by being in contact with the gastric juice within the stomach. In addition, it is possible to obtain excellent adhesion between the collapse-controlling layer 12a and adjacent layers thereof (the medicine-containing layer 11 and the gel-forming layer 13a).

Furthermore, a weight-average molecular weight of the stomach-soluble polymer is not particularly limited, but is preferably in the range of 10,000 to 1,000,000 and more preferably in the range of 30,000 to 300,000. This makes it possible to enhance the stability of the shape of the collapse-controlling layer 12a. Furthermore, it is possible for the collapse-controlling layer 12a to quickly be dissolved by being in contact with the gastric juice within the stomach. In addition, it is possible to obtain excellent adhesion between the collapse-controlling layer 12a and adjacent layers thereof (the medicine-containing layer 11 and the gel-forming layer 13a).

Furthermore, an amount of the stomach-soluble material contained in the collapse-controlling layer 12a is preferably in the range of 1 to 90 mass% and more preferably in the range of 3 to 80 mass%. This makes it possible for the collapse-controlling layer 12a to reliably be collapsed in the stomach, while exhibiting sufficiently excellent adhesion between the respective layers.

Furthermore, the collapse-controlling layer 12a, may include a base (namely, a base agent for the collapse-controlling layer). This makes it possible to reliably maintain the shape of the collapse-controlling layer 12a and improve the adhesion between the medicine-containing layer 11 and the gel-forming layer 13a. When the orally-administered agent 1a is swallowed, it is possible to reliably prevent peeling from occurring between the gel-forming layer 13a and the collapse-controlling layer 12a and between the medicine-containing layer 11 and the collapse-controlling layer 12a.

Examples of the base used in the collapse-controlling layer 12a include, but are not particularly limited to: starch such as α-starch, oxidized starch, carboxymethyl starch sodium, hydroxypropyl starch, dextrin and dextran; derivatives of starch; sugars such as saccharose, maltose, lactose, glucose, fructose, pullulan, xanthane gum, cyclodextrin, xylitol, mannitol and sorbitol; polyvinyl acetate; polyvinyl alcohol; polyvinyl pyrrolidone; a vinylacetate-vinyl pyrrolidone copolymer; natural rubbers such as Arabic gum and tragacanth gum; polyglucosamines such as chitin and chitosan; proteins such as gelatin, casein and soybean protein; titanium oxide; calcium monohydrogen phosphate; calcium carbonate; talc; stearate; magnesium aluminometasilicate; magnesium silicate; and silicic anhydride. One or more of these bases may be used independently or in combination according to the purposes of including the base into the collapse-controlling layer 12a.

Furthermore, the collapse-controlling layer 12a may include other components than the materials described above. For example, the collapse-controlling layer 12a may include: an antiseptic agent such as methyl hydroxybenzoate and propyl hydroxybenzoate; a coloring agent such as an edible lake pigment; a plasticizer such as triethyl citrate, glycerin and propylene glycol; a masking agent such as sugars; and the like.

Furthermore, it is preferred that it is difficult for the collapse-controlling layer 12a to be collapsed in other body cavities than the stomach. Concretely, it is preferred that it is difficult for the collapse-controlling layer 12a to be collapsed in the oral cavity. Therefore, it is possible to reliably prevent the medicine contained in the medicine-containing layer 11 from being released into other body cavities than the stomach by collapse of the collapse-controlling layer 12a. As a result, the orally-administered agent 1a can release the medicine reliably into the stomach.

More specifically, it is preferred that the collapse-controlling layer 12a is not collapsed within three minutes by an artificial saliva of 37°C, more preferred that it is not collapsed within five minutes and even more preferred that it is not collapsed within ten minutes. Such an artificial saliva is a liquid in which NaCl of 0.08 mass%, KCl of 0.12 mass%, MgCl₂ of 0.01 mass%, CaCl₂ of 0.01 mass%, K₂HPO₄ of 0.03 mass% and CMC-Na of 0.10 mass% are added to purified water.

More specifically, the collapse-controlling layer 12a is preferably collapsed within less than twenty minutes with respect to a first test liquid (pH: 1.2) of 37°C used in a collapse test defined in Japanese Pharmacopoeia, more preferably less than ten minutes and even more preferably less than three minutes. Therefore, the collapse-controlling layer 12a can be collapsed by the gastric juice (hydrochloric acid aqueous) within the stomach. For these reasons, the medicine contained in the medicine-containing layer 11 is reliably dissolved and released into the stomach. This makes it possible to reliably prevent the collapse-controlling layer 12a from being collapsed by the saliva within the oral cavity. Accordingly, it is possible to reliably prevent the medicine contained in the medicine-containing layer 11 from being dissolved and released into the oral cavity.

Furthermore, a thickness of the collapse-controlling layer 12a is not particularly limited, but is preferably in the range of 1 to 200 µm and more preferably in the range of 5 to 100 µm. This makes it possible for the orally-administered agent 1a to reliably release the medicine into the stomach.

### <Gel-forming Layer>

The gel-forming layer 13a is provided on the upper surface of the collapse-controlling layer 12a. On the other hand, the gel-forming layer 13b is provided on the lower surface of the collapse-controlling layer 12b.

Hereinafter, only the gel-forming layer 13a will be representatively described below, because the gel-forming layers 13a and 13b have substantially the same configuration.

The gel-forming layer 13a is a layer that can be swelled and gelatinized by absorbing water. As described above, the gel-forming layer 13a absorbs water contained in the saliva rapidly within the oral cavity, thereby forming a gel. By doing so, softness is rapidly given to the orally-administered agent 1a in the oral cavity, so that it becomes possible to swallow the orally-administered agent 1a with ease.

Even if the collapse-controlling layer 12a contains a material to adhere to the body cavity with ease, it is possible to prevent the collapse-controlling layer 12a from being in contact with an inside wall of the body cavity by providing the gel-forming layer 13a on the upper surface (outside) of the collapse-controlling layer 12a of the orally-administered agent 1a. In other words, it is possible to prevent the orally-administered agent 1a from being in contact with the inside wall of the body cavity. Generally, in the case where an orally-administered agent adheres to a inside wall of a body cavity, it is difficult for the orally-administered agent to reach intended parts (stomach) of a living body. In other words, it is difficult to release the medicine at the intended parts (within the stomach) of the living body. However, since the orally-administered agent 1a has the gel-forming layer 13a, such problems are solved reliably.

Furthermore, the gel-forming layer 13a can adjust an amount of water to be in contact with the collapse-controlling layer 12a. That is, the gel-forming layer 13a prevents a large quantity of water from being in contact with the collapse-controlling layer 12a within the oral cavity, thereby preventing the collapse-controlling layer 12a from being collapsed. On the other hand, the gel-forming layer 13a is collapsed in the stomach and reliably removed from the orally-administered agent 1a. Accordingly, the collapse-controlling layer 12a can be sufficiently in contact with the gastric juice within the stomach to thereby be collapsed. As a result, the orally-administered agent 1a can reliably release the medicine into the stomach.

The gel-forming layer 13a contains a gel-forming agent that can be swelled and gelatinized by absorbing water. The gel-forming layer 13a containing such a gel-forming agent can form a gel by easily and rapidly absorbing water existing around the gel-forming layer 13a. Furthermore, the gel-forming agent can adjust an amount and property (pH etc.) of water to be in contact with the collapse-controlling layer 12a, so that the collapse-controlling layer 12a can be reliably collapsed at the parts of the body cavity.

Examples of the gel-forming agent include: an anionic polymer; polysaccharide (starch and the like); protein (collagen, casein and the like); and the like. The anionic polymer is a polymer having anionic groups or salts thereof. The anionic groups contain carboxyl groups, sulfonic groups, phosphate groups and the like. The anionic groups are preferably the carboxyl groups. These anionic groups may include a base neutralized by salifiable cations. To be Concrete, examples of the anionic polymer include: a polymer containing the carboxyl groups such as polyacrylic acid, polymethacrylic acid, polyitaconic acid, a carboxy vinyl polymer, carboxymethyl cellulose, carboxymethyl-hydroxyethyl cellulose, alginic acid, heparin, hyaluronic acid, carrageenan, and pectinic acid; salts of these polymers; a polymer containing the sulfonic groups such as polystyrene sulfonate, polyethylene sulfonate, and polyvinyl sulfonate; salts of these polymers; and the like. In this regard, examples of the salifiable cations include a sodium cation, a potassium cation, a monoethanolamine cation, a diethanolamine cation, a triethanolamine cation, an ammonium cation and the like. One or more of these cations can be used independently or in combination.

Among these substances, it is preferred that the gel-forming agent includes the anionic polymer. The anionic polymer can rapidly absorb water and can form the gel in a rapid manner. In addition, the anionic polymer is a component relatively hard to dissolve after formation of the gel. Therefore, in the case where the anionic polymer is used as the gel-forming agent, the gel-forming layer 13a is reliably kept in a gelatinized shape within the oral cavity even after the formation of the gel. It is possible to reliably control a collapsing time of the collapse-controlling layer 12a and the parts of the body cavities at which the medicine is released. Furthermore, in the case where the polymer containing the carboxyl groups in the anionic polymer is used, the effects as described above are exhibited more conspicuously.

In such a case, a viscosity at 20°C of an aqueous solution of 0.2 mass% of the anionic polymer is preferably in the range of 1500 to 50000 mPa·s and more preferably in the range of 10000 to 20000 mPa·s. This enables the gel-forming layer 13a to rapidly absorb the water and to form the gel in the rapid manner. The gel-forming layer 13a is reliably kept in the gelatinized shape.

In the case where the anionic polymer is used as the gel-forming agent, it may be possible to cross-link the anion polymer through the use of a cross-linking agent. This ensures that the gelatinized gel-forming layer 13a is surely prevented from dissolution within the oral cavity.

The cross-linking can be performed by the cross-linking agent that varies with a kind of molecules to be cross-linked. In the case where the polymer containing the carboxyl groups is used as the anionic polymer, as the cross-linking agent for cross-linking the polymer containing the carboxyl groups, e.g., a polyvalent metal compound can be used. The polyvalent metal compound cross-links the polymer containing the carboxyl groups as follows: When the gel-forming agent contained in the gel-forming layer 13a, that is, the polymer containing the carboxyl groups is swelled and gelatinized within the oral cavity of the patient by the water contained in saliva etc., the polyvalent metal compound is ionized to thereby generate a polyvalent metal ion. Then, the polyvalent metal ion cross-links the polymer containing the carboxyl groups contained in the gel-forming layer 13a. Accordingly, even if the sufficiently cross-linked gel-forming agent is not contained in the gel-forming layer 13a preliminarily, a gel having great enough strength is formed in the gel-forming layer 13a. On the other hand, the polyvalent metal ion is easily eluted from the gel-forming layer 13a into the stomach with the gastric juice, thereby enabling the gel to be collapsed reliably. As a result, the collapse-controlling layer 12 is easily collapsed by reliably being in contact with the gastric juice within the stomach. If the collapse-controlling layer 12 is collapsed, the medicine-containing layer 11 is in contact with the gastric juice, so that the medicine contained therein is reliably released into the stomach.

Examples of the polyvalent metal compound include, but are not particularly limited to, calcium chloride, magnesium chloride, aluminum chloride, aluminum sulfate, aluminum potassium sulfate, ferric chloride alum, ammonium alum, ferric sulfate, aluminum hydroxide, aluminum silicate, aluminum phosphate, iron citrate, magnesium oxide, calcium oxide, zinc oxide and zinc sulfate. One or more of these compounds can be used independently or in combination. Use of a trivalent metal compound among these compounds makes it possible to increase a cross-linking degree of the polymer containing the carboxyl groups and enhancing physical strength of the gel-forming layer 13a. In addition, it is possible to reliably prevent the polymer containing the carboxyl groups from being dissolved within the oral cavity. Among the polymers containing the carboxyl groups cross-linked by the cross-linking agent as described above, the cross-linked polyacryl acid is preferable due to excellent gel-forming property.

A content of the gel-forming agent in the gel-forming layer 13a is preferably in the range of 5 to 90 mass% and more preferably in the range of 15 to 70 mass%, although it can be suitably adjusted depending on a kind of gel-forming agent or other factors. This enables the gel-forming layer 13a to rapidly absorb water. Furthermore, the gel-forming agent is reliably prevented from being dissolved within the oral cavity after gelatinization of the gel-forming agent.

In the case where the cross-linking agent is contained in the gel-forming layer 13a, a content of the cross-linking agent in the gel-forming layer 13a is preferably in the range of 0.1 to 2.5 mass% and more preferably in the range of 0.5 to 1.2 mass%. This makes it possible to surely prevent dissolution of the gel-forming layer 13a in the oral cavity while easily keeping the gel-forming layer 13a in the gelatinized shape after the gel-forming agent is gelatinized. In addition, it is possible to reduce a viscosity of a coating solution used as a raw material of the gel-forming layer 13a in the below-mentioned process of producing the orally-administered agent 1a, which makes it possible to efficiently form the gel-forming layer 13a.

The gel-forming layer 13a may contain a base (namely, a gel-forming base agent) which is a component contributing to stabilization of the shape of the gel-forming layer 13a. In other words, the base imparts a suitable degree of flexibility to the gel-forming layer 13a before the gel-forming layer 13a is swelled by water. Accordingly, the inclusion of the base to the gel-forming layer 13a makes it possible to prevent the orally-administered agent 1a from being cracked or damaged by an external force or other causes. After the gel-forming layer 13a has absorbed the water, the base serves to reliably keep the gel-forming layer 13a in the gelatinized shape, and prevent the formed gel from quickly collapsing.

Examples of the base used in the gel-forming layer 13a include, but are not particularly limited to, polyvinyl alcohol, polyvinyl pyrrolidone, polyvinyl acetate, polyvinylphthalate acetate, hydroxyalkyl cellulose (e.g., hydroxypropyl cellulose, hydroxypropylmethyl cellulose, hydroxymethyl cellulose or hydroxyethyl cellulose), alkyl cellulose (e.g., methyl cellulose or ethyl cellulose), (metha)acrylate and the like. One or more of these compounds can be used independently or in combination.

In the case where the base is contained in the gel-forming layer 13a, a content of the base in the gel-forming layer 13a is preferably in the range of 20 to 85 mass% and more preferably in the range of 30 to 80 mass%.

It is preferred that the base contained in the gel-forming layer 13a is water-soluble. If the base is water-soluble, it becomes easy for water to get into the gel-forming layer 13a, thereby enabling the gel-forming layer 13a to be rapidly swelled and gelatinized within the oral cavity.

Examples of the water-soluble base include: polyvinyl alcohol; hydroxyalkyl cellulose such as hydroxypropyl cellulose, hydroxypropylmethyl cellulose, methyl cellulose or the like; polyvinyl pyrrolidone; and the like.

In particular, in the case where polyvinyl alcohol is included in the base contained in the gel-forming layer 13a, the polyvinyl alcohol prevents the medicine contained in the medicine-containing layer 11 from eluting into the oral cavity. The polyvinyl alcohol can suppress the taste or order of the medicine contained in the medicine-containing layer 11 from being released into the oral cavity. That is to say, the polyvinyl alcohol can also serve as a masking agent to be described later.

Furthermore, the gel-forming layer 13a may contain a water absorption promoter for promoting water absorption of the gel-forming layer 13a. If the gel-forming layer 13a contains the water absorption promoter, it becomes possible to sufficiently increase the water absorption speed of the gel-forming layer 13a within the oral cavity. Accordingly, it becomes possible to improve the swallowability of the orally-administered agent 1a.

As the water absorption promoter, it is possible to use, e.g., a component having relatively high water-solubility. This component having relatively high water-solubility is dissolved in water and therefore can transport water into the gel-forming layer 13a. Consequently, the gel-forming layer 13a can absorb water quickly.

When an aqueous solution of 5 mass% of the water absorption promoter is prepared, a viscosity at 37°C of the aqueous solution is preferably in the range of 0.3 to 5.0 mPa·s, more preferably in the range of 0.5 to 3.5 mPa·s and even more preferably in the range of 0.6 to 1.8 mPa·s. As an indicator of water solubility of the water absorption promoter, it is possible to use, e.g., the viscosity of the aqueous solution in which the water absorption promoter is dissolved. It is possible to think that the water solubility of the water absorption promoter is improved as the viscosity of the aqueous solution grows low. That is, if the viscosity of the aqueous solution of 5 mass% of the water absorption promoter falls within the range noted above, the water absorption promoter has suitably a high degree of the water solubility within the oral cavity. This makes it possible to suitably increase the water absorption speed of the gel-forming layer 13a containing the gel-forming agent. Furthermore, this also makes it possible to surely prevent the water absorption promoter from being suddenly dissolved and dispersed into the saliva.

Examples of the water absorption promoter include, but are not particularly limited to: glycols such as propylene glycol, polyethylene glycol, polypropylene glycol, polyoxyl stearate, polyoxyethylene polyoxypropylene glycol, polyoxyethylene-cured castor oil and the like; glycerin; and sugars such as erythritol, sorbitol, xylitol, mannitol, inositol, maltitol, lactitol, glucose, xylose, mannose, fructose, galactose, sucrose, fructose, saccharose and the like. One or more of these compounds can be used independently or in combination.

It is preferred that the water absorption promoter contains glycerin among the compounds listed above. Glycerin is a component that has increased capability to promote the water absorption of the gel-forming layer 13a and has a function of being capable of imparting softness to the gel-forming layer 13a. Therefore, the orally-administered agent 1a has a suitable degree of flexibility until it is administered to a patient, which means that the orally-administered agent 1a is hardly broken or damaged by an external force. After the orally-administered agent 1a is administered to the patient, the gel-forming layer 13a becomes soft within the oral cavity while keeping its shape unchanged due to functions of glycerin described above. Thus, the orally-administered agent 1a becomes easy to swallow. Furthermore, even if the medicine contained in the medicine-containing layer 11 is eluted from the medicine-containing layer 11 into the oral cavity, glycerin is a component of be capable of suppressing uncomfortable feeling by the bitter taste of the medicine contained in the medicine-containing layer 11 within the oral cavity due to the sweet taste of glycerin.

A content of glycerin in the water absorption promoter is preferably in the range of 35 to 95 mass% and more preferably in the range of 40 to 90 mass%. This can increase the water absorption speed of the gel-forming layer 13a. Furthermore, this ensures that the orally-administered agent 1a becomes soft and easy to swallow.

Furthermore, it is preferred that the water absorption promoter contains a solid-state compound in an atmosphere of 1 atm at 25°C. Although the water absorption promoter is a component having relatively high water solubility, the addition of the solid-state compound makes it possible to prevent the orally-administered agent 1a from absorbing moisture during storage thereof. Therefore, it is possible to surely prevent the orally-administered agent 1a from being degraded during the storage thereof and to prevent the gel-forming layer 13a from being inadvertently gelatinized. Particularly, in the case where glycerin is contained in the water absorption promoter, the solid-state compound can prevent glycerin from flowing out (bleeding) during the storage of the orally-administered agent 1a.

Examples of such a solid-state compound include the glycols stated above and the afore-mentioned sugars excepting glycerin.

It the case where the water absorption promoter contains the sugars, it becomes possible to attain the following advantageous effects. More specifically, the sugars can serve as a masking agent to be described later, because they taste sweet and have increased capability to promote the water absorption of the gel-forming layer 13a. Furthermore, the sweet taste of the sugars felt within the oral cavity by a patient helps accelerate secretion of the saliva. As a result, the orally-administered agent 1a shows increased swallowability. Furthermore, the sugars and glycerin are similar in a chemical structure thereof, and therefore they have an extremely high affinity with respect to each other. Accordingly, if the water absorption promoter contains the sugars and glycerin, the sugars are capable of reliably holding glycerin in the gel-forming layer 13a and surely preventing glycerin from flowing out (bleeding) from the orally-administered agent 1a when storing the orally-administered agent 1a.

In the case where the water absorption promoter contains glycols among the compounds described above, it becomes possible to attain the following advantageous effects. Since the glycols show a good affinity to water and have a chain-like structure in a chemical structure thereof, glycols are a component that can be easily intertwined to molecules of glycols in themselves or other molecules than the glycols contained in the gel-forming layer 13a. Therefore, the glycols are linked to other molecules in the gel-forming layer 13a, thus maintaining the shape of the gel-forming layer 13a. This ensures that the gel-forming layer 13a is gelatinized with great ease while maintaining the shape of the gel-forming layer 13a. As a result, the orally-administered agent 1a shows especially high swallowability.

A content of the water absorption promoter in the gel-forming layer 13a is preferably in the range of 1 to 20 mass% and more preferably in the range of 3 to 17 mass%. This makes it possible to greatly increase the water absorption speed of the gel-forming layer 13a, while keeping the gel-forming layer 13a in a desired gel shape within the oral cavity.

Furthermore, the gel-forming layer 13a may contain a plasticizer. By containing the plasticizer, a proper degree of the softness is imparted to the gel-forming layer 13a. Examples of the plasticizer include glycerin triacetate, diethyl phthalate, triethyl citrate and lauric acid, one or more of which can be used independently or in combination.

The gel-forming layer 13a may contain a masking agent capable of suppressing the uncomfortable feeling by the taste or odor of the medicine contained in the medicine-containing layer 11. By containing the masking agent into the gel-forming layer 13a, it is possible to enhance the effect of suppressing the uncomfortable feeling by the taste or odor of the medicine contained in the medicine-containing layer 11 (what is called a masking effect), even if the medicine contained in the medicine-containing layer 11 is eluted from the medicine-containing layer 11 to the oral cavity. Examples of the masking agent include: acidic-taste imparting agents such as citric acid, tartaric acid, fumaric acid and the like; sweetening agents such as saccharin, glycyrrhizinic acid and the like; mouth fresheners such as menthol, mentha oil, peppermint, spearmint and the like; natural or synthetic perfumes; and the like. One or more among these compounds can be used independently or in combination. The afore-mentioned sugars as the water absorption promoter have a sweet taste and can serve as the masking agent.

The gel-forming layer 13a may contain other components than mentioned above. For example, the gel-forming layer 13a may contain: antiseptic agents such as methyl hydroxybenzoate, propyl hydroxybenzoate and the like; and coloring agents such as edible lake pigment and the like.

A thickness of the gel-forming layer 13a is preferably in the range of 10 to 1000 µm and more preferably in the range of 15 to 500 µm, although it may be suitably adjusted within an orally-administrable range of the orally-administered agent 1a.

### <Antiadhesive Layer>

The antiadhesive layer 14a is laminated on an upper surface of the gel-forming layer 13a at request. Such an antiadhesive layer 14a is provided as a surface layer constituting one surface of the orally-administered agent 1a. On the other hand, the antiadhesive layer 14b is laminated on a lower surface of the gel-forming layer 13b. Such an antiadhesive layer 14b is provided as a surface layer constituting the other surface of the orally-administered agent 1a.

Furthermore, the antiadhesive layers 14a and 14b are rapidly dissolved by water contained in saliva within the oral cavity and have a function of preventing the orally-administered agent 1a from adhering to the inside wall in the oral cavity. In other words, when the orally-administered agent 1a is taken in the oral cavity, surface parts of the antiadhesive layers 14a and 14b are quickly dissolved by the saliva, thereby rapidly forming liquid-state films between the antiadhesive layers 14a and 14b and the inside wall of the oral cavity, respectively. As a result, it becomes easy for the orally-administered agent 1a to slide with respect to the inside wall. Therefore, the orally-administered agent 1a is prevented from being in contact with the inside wall of the oral cavity, so that it becomes difficult to adhere to the inside wall of the oral cavity. Furthermore, even if parts of the orally-administered agent 1a adhere to the inside wall, it becomes easy for the orally-administered agent 1a to peel off from the inside wall of the oral cavity. This makes it possible to prevent uncomfortable feelings from being brought by allowing the orally-administered agent 1a to adhere to the inside wall of the oral cavity, so that it becomes easy to swallow the orally-administered agent 1a. Furthermore, it is possible to reliably transfer the medicine contained in the medicine-containing layer 11 to the intended parts of the living body.

Particularly, the orally-administered agent 1a has the gel-forming layers 13a and 13b. Therefore, the orally-administered agent 1a becomes soft in the oral cavity. This makes it possible for the orally-administered agent 1a to more easily peel off from the inside wall by deformation of the orally-administered agent 1a with weak power, even if the parts of the orally-administered agent 1a adhere to the inside wall of the oral cavity.

Hereinafter, since the antiadhesive layers 14a and 14b are substantially identical with each other in configurations thereof, a description will be made on the antiadhesive layer 14a as representative.

The antiadhesive layer 14a is rapidly dissolved by water contained in saliva etc. within the oral cavity, and is mainly constituted of an antiadhesive agent which is capable of forming a aqueous-solution-state film around the orally-administered agent 1a.

Furthermore, in the antiadhesive agent as described above, a viscosity at 37°C of an aqueous solution of 5 mass% of the antiadhesive agent is preferably 50 mPa·s or less, and more preferably 40 mPa·s or less. As an indicator of adhesive property of the antiadhesive layer 14a with respect to the inside wall of the oral cavity, it is possible to use the viscosity of the aqueous solution in which a component (antiadhesive agent) constituting the antiadhesive layer 14a is dissolved. That is, the component constituting the antiadhesive layer 14a is quickly dissolved in water in the oral cavity and therefore' an aqueous-solution-state film having a low viscosity is formed around the orally-administered agent la with ease as the viscosity of the aqueous solution grows low.

In the antiadhesive layer 14a, the phrase "mainly constituted of the antiadhesive agent" means that a concentration of the antiadhesive agent contained in the antiadhesive layer 14a is 50 mass% or higher. A content of the antiadhesive agent in the antiadhesive layer 14a is preferably 50 mass% or higher, and more preferably 70 mass% or higher. This makes it possible to conspicuously obtain the effects as described above.

The antiadhesive agent is not particularly limited as long as the viscosity characteristics as described above, that is, the viscosity at 37°C of the aqueous solution of 5 mass% of the antiadhesive agent falls within the range as described above. Examples of the antiadhesive agent include; a water-soluble polymer material such as hydroxyalkyl cellulose, polyethylene glycol, polypropylene glycol, polyvinyl alcohol, polyoxyl stearate, polyoxyethylene polyoxypropylene glycol, polyoxyethylene-cured castor oil, gum arabic, gelatin and the like; sugars such as erythritol, sorbitol, xylitol, mannitol, inositol, maltitol, lactitol, glucose, xylose, mannose, fructose, galactose, sucrose, fructose, saccharose and the like; propylene glycol; glycerin; and the like. One or more of these compounds can be used independently or in combination. Furthermore, examples of the hydroxyalkyl cellulose include hydroxypropyl cellulose, hydroxypropylmethyl cellulose, methyl cellulose and the like.

Among the antiadhesive agents described above, it is preferred that the antiadhesive layer 14a includes the water-soluble polymer material as the antiadhesive agent. The water-soluble polymer material can be reliably dissolved in water and has a molecular chain having the appropriate length in a chemical structure thereof. Therefore, the molecular chain of the water-soluble polymer material in itself can be intertwined. Furthermore, in the case where the antiadhesive layer 14a includes a plurality of kinds of water-soluble polymer materials having different solubilities, the molecular chains of the water-soluble polymer materials having the different solubilities can be appropriately intertwined to each other. Therefore, when the water-soluble polymer materials dissolve from the antiadhesive layer 14a, it is possible to reliably allow the water-soluble polymer materials to unevenly exist around the orally-administered agent 1a in a state of an aqueous solution. For this reason, the orally-administered agent 1a is reliably prevented from adhering to the inside wall of the oral cavity for a long period of time. Furthermore, the water-soluble polymer material as described above can also serve as a base of the antiadhesive layer 14a, which is possible to produce the orally-administered agent 1a with ease. In addition, it is possible for the orally-administered agent 1a to reliably exhibit superior durability when storing the produced orally-administered agent 1a. In particularly, among the water-soluble polymer materials, in the case where at least one of polyethylene glycol, hydroxypropyl cellulose and polyvinyl alcohol is used, it is possible to conspicuously exhibit the effects as described above.

A mass-average molecular weight of the water-soluble polymer material as described above is preferably in the range of 5000 to 150000 and more preferably in the range of 10000 to 100000. This makes it possible for the water-soluble polymer material to sufficiently improve solubility with respect to water. After the water-soluble polymer material is dissolved, it is possible to reliably allow the water-soluble polymer material to unevenly exist around the orally-administered agent 1a in a state of an aqueous solution. Therefore, the orally-administered agent 1a is reliably prevented from reliably adhering to the inside wall of the oral cavity for a longer period of time.

Further, among the antiadhesive agents described above, the sugars may be included in the antiadhesive layer 14a as the antiadhesive agent. The sugars are capable of serving as a masking agent to mask tastes or odor of the medicine. Furthermore, the sugars are components for accelerating secretion of the saliva in the oral cavity. Since the sugars have superior solubility with respect to water, the sugars not only serve as the antiadhesive agent but also have functions of helping that the gel-forming layer 13a is in contact with water.

Furthermore, the antiadhesive layer 14a may contain any components other than the components described above. For example, the antiadhesive layer 14a may include a plasticizer, a masking agent, an antiseptic agent, a coloring agent and the like as described above.

Furthermore, a mass of the antiadhesive layer 14a per unit area thereof is preferably in the range of 3 to 20 g/m² and more preferably in the range of 5 to 18 g/m². This makes it possible to allow an aqueous solution containing a component which has been eluted from the antiadhesive layer 14a to unevenly exist around the orally-administered agent 1a for a long period of time, while providing efficiently a thin antiadhesive layer 14a (the orally-administered agent 1a).

The orally-administered agent 1a can be produced according to, e.g., the following processes.

### (Antiadhesive Layer Production Step)

Prepared first is a coating solution (namely, a coating solution for the antiadhesive layer) containing constituent materials of the antiadhesive layer 14a.

The coating solution for the antiadhesive layer can be prepared by dispersing or dissolving the constituent materials of the antiadhesive layer 14a as described above in a liquid medium such as purified water, ethanol or the like.

Next, the coating solution for the antiadhesive layer is applied or sprayed on a supporting substrate and then dried. This produces an antiadhesive layer to become the antiadhesive layer 14a on the supporting substrate. In this regard, it is to be noted that an antiadhesive layer to become the antiadhesive layer 14b can be also formed in the same manner as the process of producing the antiadhesive layer to become the antiadhesive layer 14a.

As the supporting substrate, it is possible to use, e.g., a glass plate, a plastic film or a release sheet, but is not limited to them.

### (Gel-forming Layer production Step)

Prepared next is a coating solution (namely, a coating solution for the gel-forming layer) containing constituent materials of the gel-forming layer 13a.

The coating solution for the gel-forming layer can be prepared by dispersing or dissolving the constituent materials of the gel-forming layer 13a as described above in a liquid medium such as purified water, ethanol or the like.

Next, the coating solution for the gel-forming layer is applied or sprayed on the antiadhesive layer formed on the supporting substrate and then dried. This produces a gel-forming layer to become the gel-forming layer 13a on the antiadhesive layer. In this regard, it is to be noted that a gel-forming layer to become the gel-forming layer 13b can be also formed in the same manner as the process of producing the gel-forming layer to become the gel-forming layer 13a.

### (Collapse-Controlling Layer production Step)

Prepared next is a coating solution (namely, a coating solution for the collapse-controlling layer) containing constituent materials of the collapse-controlling layer 12a.

The coating solution for the collapse-controlling layer can be prepared by dispersing or dissolving the constituent materials of the collapse-controlling layer 12a as described above in a liquid medium such as purified water, ethanol or the like.

Next, the coating solution for the collapse-controlling layer is applied or sprayed on the gel-forming layer formed on the antiadhesive layer on the supporting substrate and then dried. This produces a gel-forming layer to become the collapse-controlling layer 12a. In this regard, it is to be noted that a gel-forming layer to become the collapse-controlling layer 12b can be also formed in the same manner as the process of producing the gel-forming layer to become the collapse-controlling layer 12a.

### (Intermediate Body Production Step)

Prepared next is a coating solution (namely, a coating solution for the medicine-containing layer) containing constituent materials of the medicine-containing layer 11.

The coating solution for the medicine-containing layer can be prepared by dispersing or dissolving the constituent materials of the medicine-containing layer 11 as described above in a liquid medium such as purified water, ethanol or the like.

Next, the coating solution for the medicine-containing layer is applied or sprayed on each of the collapse-controlling layers and then dried. This produces a precursor of the medicine-containing layer (namely, a precursor for the medicine-containing layer) on the collapse-controlling layer. In other words, an intermediate body for the orally-administered agent (hereinafter, simply referred to as an intermediate body) consisting of the precursor for the medicine-containing layer, the collapse-controlling layer, the gel-forming layer and the antiadhesive layer is produced. In this regard, the coating solution for the medicine-containing layer is applied on only parts of the collapse-controlling layer on which the medicine-containing layer is provided. The coating solution for the medicine-containing layer is not applied on other parts of the collapse-controlling layer. Another intermediate body is produced by the same process as that described above.

### (Thermal Compression Bonding Step)

Next, the two intermediate bodies produced in the intermediate body production step are thermally fusion-bonded together under a pressure so that the precursors of the medicine-containing layers of the intermediate bodies can be bonded to each other. Thus, the precursors of the two medicine-containing layers are fusion-bonded to form a single medicine-containing layer 11. Furthermore, the medicine-containing layer 11 is covered with the collapse-controlling layers 12a and 12b. In view of the above, obtained is the orally-administered agent 1a constituted from the laminate body which consists of the two antiadhesive layers 14a and 14b, the two gel-forming layers 13a and 13b, the two collapse-controlling layers 12a and 12b and the medicine-containing layer 11. The laminated body may be used as the orally-administered agent 1a as it stands, or may be processed by a method of punching it into an arbitrary shape, such as a circular shape, an elliptical shape or a polygonal shape, to produce the orally-administered agent 1a.

Furthermore, the orally-administered agent 1a may be produced by, e.g., repeating the tasks of applying and drying the coating solution for the antiadhesive layer, the coating solution for the gel-forming layer, the coating solution for the collapse-controlling layer and the coating solution for the medicine-containing layer as described above.

### <Second Embodiment>

Next, a description will be made on an orally-administered agent in accordance with a second embodiment of the present invention.

FIG. 2 is a section view showing an orally-administered agent in accordance with a second embodiment.
Hereinafter, the orally-administered agent in accordance with the second embodiment of the present invention will now be described with reference to FIG. 2. The following description will be centered on the points differing from the first embodiment, with the same items omitted from the description.

As shown in FIG. 2, the orally-administered agent 1b of the present embodiment differs from that of the first embodiment in that surfaces of gel-forming layers 13c and 13d defining outer surfaces of the orally-administered agent 1b have a plurality of convex portions 131 (hereinafter, simply referred to as "convex portions 131") and the antiadhesive layers 14a and 14b are not provided.

Provision of the convex portions 131 on the surfaces of the gel-forming layers 13c and 13d as outermost surfaces of the orally-administered agent 1b makes it possible to reduce a contact area between the inside wall of the oral cavity and the orally-administered agent 1b. As a result, the orally-administered agent 1b is reliably prevented from adhering to the inside wall within the oral cavity. Therefore, it becomes easy for the medicine to reach the intended parts of the living body. In addition, provision of the convex portions 131 on the surfaces of the gel-forming layers 13c and 13d makes it possible to greatly increase a speed at which the gel-forming layers 13c and 13d absorb water from the saliva within the oral cavity. In other words, a contact area between the saliva and each of the gel-forming layers 13c and 13d can be increased by forming the convex portions 131, which results in a sharp increase in the water absorption speed of the gel-forming layers 13c and 13d. Thanks to the features noted above, the orally-administered agent 1b exhibits especially high swallowability.

The pitch p between the convex portions 131 of each of the gel-forming layers 13c and 13d is not particularly limited, but may be preferably in the range of 100 to 1,000 µm and more preferably in the range of 250 to 750 µm. This surely prevents the orally-administered agent 1b from adhering to the inside wall of the oral cavity, while greatly increasing the water absorption speed.

The width w of each of the convex portions 131 of each of the gel-forming layers 13c and 13d is not particularly limited, but may be preferably in the range of 20 to 300 µm and more preferably in the range of 50 to 250 µm. This surely prevents the orally-administered agent 1b from adhering to the inside wall of the oral cavity, while greatly increasing the water absorption speed. It is also possible to surely prevent the gel-forming layers 13c and 13d from being dissolved in the saliva.

The height d of each of the convex portions 131 of each of the gel-forming layers 13c and 13d is not particularly limited, but may be preferably in the range of 10 to 5000 µm and more preferably in the range of 20 to 1,000 µm. This surely prevents the orally-administered agent 1b from adhering to the inside wall of the oral cavity, while greatly increasing the water absorption speed. It is also possible to surely prevent the gel-forming layers 13c and 13d from being dissolved in the saliva.

The gel-forming layers 13c and 13d having the convex portions 131 set forth above can be produced by, e.g., forming, on a surface of a supporting substrate, concave portions having a pattern complementary to that of the convex portions 131 to be formed on the gel-forming layer, applying the coating solution containing the constituent materials of the gel-forming layers 13c and 13d on the supporting substrate and drying the coating solution, when the gel-forming layers 13c and 13d are produced. Alternatively, the gel-forming layers 13c and 13d having the convex portions 131 may be produced by, e.g., producing the orally-administered agent 1b in the same manner as used in the first embodiment described above and then pressing a supporting substrate, a mold or an emboss roll, which have concave portions of a pattern complementary to that of the convex portions to be formed, against the gel-forming layers 13c and 13d.

While the illustrated embodiments of the present invention have been described hereinabove, the present invention shall not be limited thereto.

For example, an orally-administered agent according to the present invention may include additional arbitrary layers formed between each of the gel-forming layers and the medicine-containing layer. Furthermore, for example, the medicine-containing layer may be formed in a powder, compressive tablet or liquid manner. Furthermore, for example, the orally-administered agent may include no antiadhesive layers. Furthermore, for example, an adhesive layer for enhancing cohesion between each of the collapse-controlling layers and the medicine-containing layer may be provided.

Furthermore, for example, antiadhesive layers may be provided on outermost surfaces of the orally-administered agent. Concave portions may be formed on such antiadhesive layers.

### Examples

Next, concrete examples of the orally-administered agent according to the present invention will be described.

### 1. Production of Orally-Administered Agent (Example 1)

### (a) Antiadhesive layer Production Step

First, a coating solution A containing constituent materials of an antiadhesive layer was prepared.

Polyvinyl alcohol (Gohsenol EG05 produced by Nippon Synthetic Chemical Industry Co., Ltd.) as an antiadhesive agent was slowly added to purified water while stirring the same to obtain a mixture A. Thereafter, the mixture A was heated to 70°C and stirred for one hour to obtain the coating solution A.

Next, the coating solution A was sufficiently defoamed. Then, the coating solution A was flat-applied on an opposite surface of a release treatment surface of a supporting substrate by using an applicator in which gaps between polyethylene terephthalate film as the supporting substrate (SP-PET3811 produced by Lintec Corp.) and a blade which the applicator had were adjusted so that an amount of an antiadhesive layer obtained after the applied coating solution A was dried became 15 g/m². Thereafter, the coating solution A thus applied was dried at 85°C for five minutes, thus producing the antiadhesive layer.

### (b) Gel-forming Layer Production Step

Next, a coating solution B containing constituent materials of a gel-forming layer was prepared.

1.5 mass parts of calcium chloride (calcium chloride defined in Japanese Pharmacopoeia and produced by Tomita Pharmaceutical Co., Ltd.) was added to 1015 mass parts of purified water. The resultant mixture was stirred sufficiently to dissolve calcium chloride. As a result, an aqueous solution of calcium chloride was obtained. Then, 56.5 mass parts of polyacrylic acid (Carbopol 974P produced by CBC Co., Ltd., a viscosity of an aqueous solution of 0.2 mass% is 12100 mPa·s) was slowly added to the aqueous solution of calcium chloride while stirring the same to obtain a mixture B. After the addition of polyacrylic acid, the mixture B was stirred for about one hour. Next, 33.9 mass parts of polyvinyl alcohol (Gohsenol EG05 produced by Nippon Synthetic Chemical Industry Co., Ltd.) was slowly added to the mixture B while stirring the same to obtain a mixture C. After the addition of polyvinyl alcohol, the mixture C to which the respective materials had added was heated to 70°C and stirred for about one hour. Next, 8.1 mass parts of glycerin (thick glycerin defined in Japanese Pharmacopoeia and produced by ADEKA Corp.) as a water absorption promoter was added to the mixture C and stirred for about ten minutes, thereby producing the coating solution B.

Next, the coating solution B was sufficiently defoamed. Then, the coating solution B was flat-applied on the antiadhesive layer produced in the step (a) by using an applicator in which gaps between a polyester terephthalate film and a blade were adjusted so that an amount of a gel-forming layer obtained after the applied coating solution B was dried became 30 g/m². Thereafter, the coating solution B thus applied was dried at 80°C for ten minutes, thus producing the gel-forming layer.

### (c) Collapse-controlling Layer Production Step

Next, a coating solution C containing constituent materials of a collapse-controlling-layer was prepared.

80 mass parts of methyl methacrylate-butyl methacrylate-dimethylaminoethyl methacrylate copolymer (produced by Rheam Phama GmbH., a molecular weight: 150,000) as a stomach-soluble material was added to 233 mass parts of ethanol to obtain a mixture D. The mixture D was stirred to dissolve methyl methacrylate-butyl methacrylate-dimethylaminoethyl methacrylate copolymer. Then, 20 mass parts of triethyl citrate was added to the mixture D and stirred for about ten minutes to obtain the coating solution C.

Next, the coating solution C was sufficiently defoamed. Then, the coating solution C was flat-applied on the gel-forming layer produced in the step (b) by using an applicator in which gaps between a polyester terephthalate film and a blade were adjusted so that an amount of a collapse-controlling layer obtained after the applied coating solution C was dried became 50 g/m². Thereafter, the coating solution C thus applied was dried at 90°C for five minutes, thus producing the collapse-controlling layer.

### (d) Intermediate Body Production Step

First, a coating solution D containing constituent materials of a medicine-containing layer was prepared.
2.5 mass parts of famotidine as a gastric ulcer medicine and 0.6 mass parts of titanium oxide (TIPAQUE CR-50 produced by Ishihara Sangyo Kaisha, Ltd.) were added to 53.7 mass parts of purified water and sufficiently dispersed through the use of a homogenizer to obtain dispersion liquid. Thereafter, 13.8 mass parts of polyvinyl pyrrolidone (PVP K-90 produced by ISP Japan Ltd.) was slowly added to the dispersion liquid while stirring the same to obtain a mixture E. After the addition of polyvinyl pyrrolidone, the mixture E was stirred for about thirty minutes. Next, 4.0 mass parts of glycerin (thick glycerin defined in Japanese Pharmacopoeia and produced by ADEKA Corp.) was added to the mixture E and stirred for about five minutes, thereby producing the coating solution D.

Next, the coating solution D was sufficiently defoamed. Then, the coating solution D was applied on the collapse-controlling layer produced in the step (c) by using a screen printing. In this regard, the coating solution D was applied on a plurality of parts on the collapse-controlling layer so that an amount of a medicine-containing layer precursor obtained after the applied coating solution D was dried became 50 g/m² and a shape of the medicine-containing layer precursor became a circular shape of which diameter was 10 mm. Thereafter, the coating solution D thus applied was dried at 80°C for five minutes, thus producing the medicine-containing layer precursor. Consequently, obtained were a laminated body (intermediate body) consisting of the medicine-containing layer precursor, the collapse-controlling layer, the gel-forming layer, and the antiadhesive layer. Another intermediate body was formed in the same process as those in (a) the medicine-containing layer production step, (b) the gel-forming layer production step, (c) the collapse-controlling layer production step, and (d) the intermediate body production step.

### (e) Thermal Compression Bonding Step

The two intermediate bodies produced in the step (d) were thermally fusion-bonded together at a temperature of 100°C, under the conditions of a pressure of 1 kgf/cm² and for one second so that the medicine-containing layer precursors could be bonded to each other. Next, the polyethylene terephthalate film was peeled off from each antiadhesive layer, thereby producing a laminated body (orally-administered agent) in which the antiadhesive layer, the gel-forming layer, the collapse-controlling layer, the medicine-containing layer, the collapse-controlling layer, the gel-forming layer, and the antiadhesive layer are laminated in this order. The laminated body was processed by punching so that a whole of the medicine-containing layer was included and a shape thereof was a circular shape having a diameter of 15 mm. Consequently, the orally-administered agent as shown in FIG. 1 was obtained. Furthermore, in the orally-administered agent, two collapse-controlling layers were bonded to each other, so that the medicine-containing layer was covered by the two collapse-controlling layers.

### (Examples 2 to 5)

In each of the Examples 2 to 5, an orally-administered agent was obtained in the same manner as in the Example 1, except that the kind and content of each of the constituent materials of the gel-forming layer, the collapse-controlling layer, and the antiadhesive layer were changed as shown in Tables 1 and 2.

### (Example 6)

An orally-administered agent was obtained in the same manner as in the Example 1, except that (a) antiadhesive layer production step was not performed and the application conditions of the coating solution B (production conditions of the gel-forming layer) were changed as mentioned below.

The coating solution B was sufficiently defoamed. Next, the coating solution B was flat-applied on a polyethylene terephthalate film (supporting substrate) by using an applicator in which gaps between a polyethylene terephthalate film as the supporting substrate and a blade were adjusted so that an amount of a gel-forming layer obtained after the applied coating solution B was dried became 20 g/m². The polyethylene terephthalate film had concave portions (having the mouth size of 450x450 µm, the depth of 30 µm and the bottom size of 184x184 µm) provided in a grid pattern at a pitch of 550 µm. Thereafter, the coating solution B thus applied was dried at 80°C for five minutes, thus producing the gel-forming layer. The gel-forming layer thus produced was provided with convex portions having the height of about 30 µm, the width of about 450 µm and the pitch of about 550 µm, the shape of which was transferred from the concave portions of the polyethylene terephthalate film.

### (Examples 7 and 8)

In each of the Examples 7 and 8, an orally-administered agent was obtained in the same manner as in the Example 6, except that the kind and content of each of the constituent materials of the gel-forming layer, and the collapse-controlling layer were changed as shown in Table 1.

### (Comparative Example 1)

An orally-administered agent was obtained in the same manner as in the Example 1, except that the antiadhesive layers and the gel-forming layers were not provided.

### (Comparative Example 2)

An orally-administered agent was obtained in the same manner as in the Example 1, except that the gel-forming layers and the collapse-controlling layers were not provided.

Tables 1 and 2 shown the constituent materials of the respective layers of the orally-administered agents obtained in the respective Examples and the respective Comparative Examples and the contents thereof. In Tables 1 and 2, the name "MMM" signifies methyl methacrylate-butyl methacrylate-dimethylaminoethyl methacrylate copolymer, the name "PVAA" signifies polyvinyl acetal diethylaminoacetate, the name "TC" signifies triethyl citrate, the name "PAA" signifies polyacrylic acid (Carbopol 974P produced by CBC Cho., Ltd.), the name "EG05" signifies polyvinyl alcohol (Gohsenol EG05 produced by Nippon Synthetic Chemical Industry Co., Ltd.), the name "EG40" signifies polyvinyl alcohol (Gohsenol EG40 produced by Nippon Synthetic Chemical Industry Co., Ltd.), the name "G" signifies glycerin (thick glycerin defined in Japanese Pharmacopoeia and produced by ADEKA Corp.), the name "Man" signifies mannitol, the name "PEG" signifies polyethylene glycol #4UUU (having the HLB of 20 and the molecular weight of 4000, which is produced by Kanto Chemical Co., Inc.), the name "PEP" signifies polyoxyethylene (105) polyoxypropylene (5) glycol (PEP-101 having the HLB of 20 and the molecular weight of 4000 to 5500, which is produced by Freund Corporation), the name "HPC" signifies hydroxypropyl cellulose (HPC(L) having the mass-average molecular weight of 60000, which is produced by NIPPON SODA CO., LTD.). Furthermore, the viscosity of the water absorption promoter in the Table 1 and the viscosity of the antiadhesive agent in the Table 2 denote a viscosity at 37°C of an aqueous solution of 5 mass% of the water absorption promoter and the antiadhesive agent, respectively. Each viscosity was measured with an E-type viscometer (a product of Tokimec, Inc.). In this regard, in the Examples 1, 5, 6 and the Comparative Example 1 in Table 1, the viscosity of the water absorption promoter denotes a viscosity of an aqueous solution of 5 mass% of glycerin, in other Examples in Table 1, the viscosity of the water absorption promoter denotes a viscosity of an aqueous solution of 5 mass% of other water absorption promoter than glycerin.

### Table 1

**Table 1**

| | Collapse-controling layer | | | | Gel-forming layer | | | | | | | | | | |
|---|---|---|---|---|---|---|---|---|---|---|---|---|---|---|---|
| | Stomach-soluble material | | Plasticizer | | Base | | Gel-forming agent | | Cross-linking agent | | Water absorption promoter | | | | |
| | Kind | Content [Mass parts] | Kind | Content [Mass parts] | Kind | Content [Mass parts] | Kind | Content [Mass parts] | Kind | Content Kind [Mass parts] | Kind | Content [Mass parts] | Kind | Content [Mass parts] | Viscosity [mPa·s] |
| Ex. 1 | MMM | 80.0 | TC | 20.0 | EG05 | 33.9 | PAA | 56.5 | CaCl₂ | 1.5 | G | 8.1 | - | - | 0. 90 |
| Ex. 2 | MMM | 80.0 | TC | 20.0 | EG40 | 66.8 | PAA | 22.2 | CaCl₂ | 0.6 | G | 7.9 | Man | 2.5 | 0. 87 |
| Ex. 3 | MMM | 80.0 | TC | 20.0 | EG05 | 66.8 | PAA | 22.2 | CaCl₂ | 0.6 | G | 7.9 | PEG | 2.5 | 1.29 |
| Ex. 4 | MMM | 80.0 | TC | 20.0 | EG05 | 66.8 | PAA | 22.2 | CaCl₂ | 0.6 | G | 7.9 | PEP | 2.5 | 1.62 |
| Ex. 5 | PVAA | 80.0 | TC | 20.0 | EG40 | 80.2 | PAA | 11.5 | CaCl₂ | 0.3 | G | 8.1 | - | - | 0.90 |
| Ex.6 | MMM | 80.0 | TC | 20.0 | EG05 | 33.9 | PAA | 56.6 | CaCl₂ | 1.5 | G | 8.1 | - | - | 0.90 |
| Ex. 7 | MMM | 80.0 | TC | 20.0 | EG05 | 66.8 | PAA | 22.2 | CaCl₂ | 0. 6 | G | 7.9 | PEP | 2.5 | 1.62 |
| EX. 8 | PVAA | 80.0 | TC | 20.0 | EG40 | 66.8 | PAA | 22.2 | CaCl₂ | 0.6 | G | 7.9 | Man | 2.5 | 0.87 |
| Comp.Ex.1 | MMM | 80.0 | TC | 20.0 | - | - | - | - | - | - | - | - | - | - | - |
| Comp.Ex.2 | - | - | - | - | - | - | - | - | | - | - | - | - | - | - |

### Table 2

**Table 2**

| | Antiadhesive layer | | | | | |
|---|---|---|---|---|---|---|
| | Antiadhesive agent | | | | | |
| | Kind | Content [Mass parts] | Viscosity [mPa·s] | Kind | Content [Mass parts] | Viscosity [mPa·s] |
| Ex. 1 | EG05 | 100 | 5.4 | - | - | - |
| Ex. 2 | EG05 | 75 | 5.4 | Man | 25 | 0.87 |
| Ex. 3 | EG05 | 75 | 5.4 | PEG | 25 | 1.29 |
| Ex. 4 | PEG | 100 | 1. 29 | - | - | - |
| Ex. 5 | HPC | 100 | 27.6 | - | - | - |
| Comp.Ex.2 | EG05 | 100 | 5.4 | - | - | - |

### 2. Evaluation of Collapse Property of Collapse-controlling Layer

In the orally-administered agent of each of the Examples and the Comparative Examples, collapse property of the collapse-controlling layers was examined according to a collapse test defined in Japanese Pharmacopoeia Fifteenth Edition. The results were evaluated by visual contact.

First, the coating solution B to form the collapse-controlling layer, which was used in the respective Examples and the respective Comparative Examples, was flat-applied on an opposite surface of a release treatment surface of a polyethylene terephthalate film (SP-PET3811 produced by Lintec Corp.) by using an applicator' in which gaps between the polyethylene terephthalate film and a blade which the applicator had were adjusted so that an amount of a collapse-controlling layer obtained after the applied coating solution B was dried became 50 g/m². Thereafter, the coating solution B thus applied was dried at 90°C tor five minutes, thereby removing the polyethylene terephthalate film to obtain a sheet consisting of the collapse-controlling layer. The sheet was treated by a punching method so that a shape thereof became circular shape having a diameter of 15 mm. By these processes, a sample to evaluate the collapse property was obtained.

An artificial saliva and a first liquid (pH: 1.2) for the collapse test were used as a test liquid used in the collapse test. Such an artificial saliva is a liquid in which NaCl of 0.08 mass%, KCl of 0.12 mass%, MgCl₂ of 0.01 mass%, CaCl₂ of 0.01 mass%, K₂HPO₄ of 0.03 mass% and CMC-Na of 0.10 mass% are added to purified water. By using these test liquids and the sample, a test was performed in the same manner as a method of handling a controlled release preparation. In the respective test liquids, time until the sample was absolutely collapsed was measured. In this regard, it is to be noted that the test was performed three times to the respective test liquids to obtain an average value of the time values which were obtained by the tests of the three times. The average value was evaluated according to fourth criteria described below.

### [Evaluation Criteria According to Collapse Property Using Artificial Saliva]

A···Time until the collapse-controlling layer was collapsed was 10 minutes or more.
B···Time until the collapse-controlling layer was collapsed was 5 minutes or more but lower than 10 minutes.
C···Time until the collapse-controlling layer was collapsed was 3 minutes or more but lower than 5 minutes.
D···Time until the collapse-controlling layer was collapsed was lower than 3 minutes.

### [Evaluation Criteria According to Collapse Property Using First Liquid for Collapse Test]

A···Time until the collapse-controlling layer was collapsed was lower than 3 minutes.
B···Time until the collapse-controlling layer was collapsed was 3 minutes or more but lower than 5 minutes.
C···Time until the collapse-controlling layer was collapsed was 5 minutes or more but lower than 20 minutes.
D···Time until the collapse-controlling layer was collapsed was 20 minutes or more.

### 3. Evaluation of Elution Property of Medicine

In the orally-administered agent of each of the Examples and the Comparative Examples, elution property of the medicine contained in the medicine-containing layer was tested according to an elution test (Paddle Method) defined in Japanese Pharmacopoeia Fifteenth Edition. The results were evaluated. The test was performed by using the artificial saliva (described above) and a first liquid (pH: 1.2) for the elution test as a test liquid. In each test liquid, time until the medicine was eluted from the medicine-containing layer to each test liquid was measured. In this regard, it is to be noted that the test was performed three times to the respective test liquids to obtain an average value of the time values which were obtained by the tests of the three times. The average value was evaluated according to fourth criteria described below.

### [Evaluation Criteria According to Elution Property Using Artificial Saliva]

A···Time until the medicine was eluted from the medicine-containing layer to the artificial saliva was 10 minutes or more.
B···Time until the medicine was eluted from the medicine-containing layer to the artificial saliva was 5 minutes or more but lower than 10 minutes.
C···Time until the medicine was eluted from the medicine-containing layer to the artificial saliva was 3 minutes or more but lower than 5 minutes.
D···Time until the medicine was eluted from the medicine-containing layer to the artificial saliva was lower than 3 minutes.

### [Evaluation Criteria According to Elution Property Using First Liquid for Elution Test]

A···Time until the medicine was eluted from the medicine-containing layer to the first liquid for the elution test was lower than 3 minutes.
B···Time until the medicine was eluted from the medicine-containing layer to the first liquid for the elution test was 3 minutes or more but lower than 5 minutes.
C···Time until the medicine was eluted from the medicine-containing layer to the first liquid for the elution test was 5 minutes or more but lower than 20 minutes.
D···Time until the medicine was eluted from the medicine-containing layer to the first liquid for the elution test was 20 minutes or more.

### 4. Evaluation of Adhesive Property

Gargling was conducted to cleanse the interior of the oral cavity. After two minutes, the orally-administered agent produced in each of the Examples and the Comparative Examples was put in the mouth without water so as to on purpose adhere to the palate with ease. Thereafter, it was confirmed whether or not each orally-administered agent adhered to the palate. In the case where each orally-administered agent adhered to the palate, it was confirmed whether or not the orally-administered agent could be peeled off from the palate by tongue. The results were evaluated according to the below five criteria. In this regard, it is to be noted that the evaluation of the adhesive property was carried out five times to obtain five values. Then, an average value was obtained from the obtained five values as an overall evaluation.

1·····A whole of one surface of the orally-administered agent adhered to the palate, and the adhered whole of the one surface could not be peeled off by tongue with ease.
2·····A part of one surface of the orally-administered agent adhered to the palate, and the adhered part of the one surface could not be peeled off by tongue with ease.
3·····Although a part or whole of one surface of the orally-administered agent adhered to the palate, the adhered part or whole of the one surface could be peeled off by tongue with ease.
4·····Although a part or whole of one surface of the orally-administered agent adhered to the palate, the adhered part or whole of the one surface could be quickly peeled off.
5·····The orally-administered agent hardly adhered to the palate.

### 5. Evaluation of Swallowability (Evaluation of Administrability)

Gargling was conducted to cleanse the interior of the oral cavity. After two minutes, each of the orally-administered agents produced in the respective Examples and the Comparative Examples was put into the oral cavity without water and swallowed. The swallowability of the orally-administered agent was evaluated according to the below five criteria. In this regard, it is to be noted that the evaluation was carried out five times to obtain five values. Then, an average value was obtained from the obtained five values as an overall evaluation. At the same time, it was confirmed whether or not the orally-administered agent stuck in the throat, the airway and the esophagus when swallowing the orally-administered agent (safety of administrating).

### [Evaluation Criteria According to Swallowability]

1···The orally-administered agent was not swelled and gelatinized and could not be administrated without water.
2···The orally-administered agent was slightly swelled and gelatinized but could not be administrated without water.
3···The orally-administered agent was swelled and gelatinized but wanted to be administrated with water if possible.
4···The orally-administered agent was slowly swelled and gelatinized but could be administrated without water.
5···The orally-administered agent was quickly swelled and gelatinized but could be administrated without water.
These results are shown in Table 3.

### Table 3

**Table 3**

| | Evaluation of collapse property of collapse-controlling layer | | Evaluation of elution property of medicine | | Evaluation of adhesive property | Evaluation of swallowability | |
|---|---|---|---|---|---|---|---|
| | Artificial saliva | First liquids for collapse test | Artificial saliva | First liquid for elution test | | Swallowability | Sticking in the throat etc. |
| Ex. 1 | A | A | A | A | 5. | 4.0 | No |
| Ex. 2 | A | A | A | A | 5.0 | 5.0 | No |
| Ex. 3 | A | A | A | A | 5.0 | 5.0 | No |
| Ex. 4 | A | A | A | A | 5.0 | 5.0 | No |
| Ex. 5 | A | A | A | A | 4.8 | 4.0 | No |
| Ex. 6 | A | A | A | A | 5.0 | 4.2 | No |
| Ex. 7 | A | A | A | A | 5.0 | 5.0 | No |
| Ex. 8 | A | A | A | A | 5.0 | 5.0 | No |
| Comp.Ex.1 | A | A | A | A | 5.0 | 1.0 | Yes |
| Comp.Ex.2 | - | - | D | D | 5.0 | 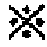 Dissolved | No |

As shown in Table 3, the orally-administered agent obtained in each of the Examples could release the medicine reliably in an acidic liquid (first liquid for the test liquid). On the other hand, it was difficult for the orally-administered agent obtained in each of the Examples to release in the artificial saliva. In other words, since the orally-administered agent obtained in each of the Examples did not release the medicine within the oral cavity, it was considered that the orally-administered agent exhibits superior property of masking tastes or odors of the medicine when swallowing. Furthermore, since it was easy for the orally-administered agent obtained in each of the Examples to dissolve to an acidic gastric juice with ease, it was considered that it was easy to release the medicine in the stomach. In particular, it was difficult for the orally-administered agents having the antiadhesive layers (the Examples 1 to 5) and the orally-administered agent having the gel-forming layers of which surface had convex portions (the Examples 6 to 8) to adhere to the inside wall of the oral cavity. As a result, it had found that it was difficult for the orally-administered agent to adhere to walls of the body cavity and it was easy to swallow the orally-administered agent. Accordingly, it was presumed that the orally-administered agent could be reliably delivered to the intended parts of the living body without adhering to the body cavity.

In contrast, in the orally-administered agent obtained in each of the Comparative Examples, no satisfactory results were obtained. In other words, the orally-administered agent of the Comparative Example 1 could not be administrated without water and stuck in the throat and the like with ease. Furthermore, the orally-administered agent of the Comparative Example 2 released the medicine in the artificial saliva with ease. Accordingly, the orally-administered agent of the Comparative Example 2 also released the medicine within the oral cavity with ease.

Furthermore, all of the water absorption promoter used in each of the Examples were solid-state compounds under the conditions of 1 atm at 25°C except for glycerin.

### EXPLANATIN OF REFERENCE NUMERAL

- 1, 1a: orally-administered agent
- 11: medicine-containing layer
- 12a, 12b: collapse-controlling layer
- 13a, 13b, 13c, 13d, 13b, 13c, 13d: gel-forming layer
- 131: convex portions
- 14b: antiadhesive layer

### INDUSTRIAL APPLICABILITY

An orally-administered agent according to the present invention can be swallowed with ease and release a medicine in the stomach. Therefore, the orally-administered agent according to the present invention can be reliably used to, in particular, aged persons or infants. Accordingly, the orally-administered agent according to the present invention contributes to the development of industries such as pharmaceutical preparations.

## Claims

1. An orally-administered agent comprising:
a medicine-containing layer containing a medicine and having surfaces;
collapse-controlling layers respectively provided on the surfaces of the medicine-containing layer; and
gel-forming layers respectively provided on the collapse-controlling layers, wherein the gel-forming layers are swelled and gelatinized by absorbing water to form a gel;
wherein the collapse-controlling layers are constituted of a material containing a stomach-soluble material to be dissolved by being in contact with gastric juice.

2. The orally-administered agent as claimed in claim 1, wherein the stomach-soluble material is a stomach-soluble polymer.

3. The orally-administered agent as claimed in claim 2, wherein the stomach-soluble polymer is a stomach-soluble acrylic acid-based copolymer.

4. The orally-administered agent as claimed in claim 1, wherein the medicine-containing layer is covered with the collapse-controlling layers.

5. The orally-administered agent as claimed in claim 1, wherein the gel-forming layers contain an anionic polymer.

6. The orally-administered agent as claimed in claim 1, wherein the orally-administered agent has surfaces and surface layers constituting the surfaces, the orally-administered agent further comprising at least one antiadhesive layer as the surface layers,
wherein the at least one antiadhesive layer prevents the orally-administered agent from adhering to an inside wall of an oral cavity by dissolving to water.

7. The orally-administered agent as claimed in claim 1, wherein each of the gel-forming layers has a surface provided with a plurality of convex portions.
